# EUROPEAN PATENT SPECIFICATION

(11) **EP 3 552 209 B1**
(45) Date of publication and mention of the grant of the patent: **12.02.2025**
(21) Application number: 17832916.5
(22) Date of filing: 05.12.2017
(51) Int. Cl.: G16H 10/60, G16H 15/00, G16H 40/63

(54) **CONTEXTUAL LIST VIEWING WITH SPARSE FEEDBACK**
KONTEXTABHÄNGIGE LISTENANSICHT MIT WENIG RÜCKMELDUNG
AFFICHAGE DE LISTES EN FONCTION DU CONTEXTE AVEC PEU DE RÉTROACTION

(30) Priority: 06.12.2016 US 201662430577 P
(43) Date of publication of application: 16.10.2019
(73) Proprietor: Koninklijke Philips N.V., 5656 AG Eindhoven (NL); The University of Chicago, Chicago, IL 60637 (US)
(72) Inventor: SEVENSTER, Merlijn, 5656 AE Eindhoven (NL); FORSBERG, Thomas, Andre, 5656 AE Eindhoven (NL); CHANG, Paul, Joseph, 5656 AE Eindhoven (NL)
(74) Representative: Philips Intellectual Property & Standards
(86) International application number: PCT/EP2017/081541
(87) International publication number: WO 2018/104312

(56) References cited:
- WO-A2-2012/031052
- US-A1- 2002 178 191
- US-A1- 2005 209 882
- US-A1- 2008 189 645
- US-A1- 2015 242 571
- US-A1- 2016 147 946
- US-B1- 7 676 469
- US-B1- 8 214 380
- US-B1- 8 527 291
- BERNAU SARAH ET AL: "Oracle Fusion Middleware User's Guide for Oracle WebCenter Spaces", 1 August 2011 (2011-08-01), pages 1 - 1912, XP093091707, Retrieved from the Internet <URL:https://web.archive.org/web/20150912143906if_/http://docs.oracle.com/cd/E25054_01/webcenter.1111/e10149.pdf> [retrieved on 20231013]

## Description

### FIELD OF THE INVENTION

The following generally relates to viewing medically related lists with a controlling lexicon, and more specifically to viewing a list of medically related data about a patient from an electronic medical record that is presented in an entirety to a variety of healthcare practitioners.

### BACKGROUND OF THE INVENTION

An electronic medical record (EMR) or other repository of medical data includes a number of lists compiled according to a controlling lexicon that are routinely viewed by a variety of healthcare practitioners in the delivery of patient care to individual patients. Examples of lists include a patient problem list, a list of patient medications, a surgical history list, a list of lab values, and the like. Each list includes an element with a plurality of occurrences. Each occurrence includes data represented according to a controlling lexicon. Examples of controlling lexicons include International classification of Diseases (ICD)-9, ICD-10, Systematized Nomenclature of Medicine (SNOMED), Current Procedural Terminology (CPT), compilations of pharmaceutical names such as a physician's desk reference, RadLex^{®} Playbook, Nursing Outcomes Classification (NOC), NANDA-I, geographical adaptations of controlling lexicons, and the like.

For example, a patient problem list includes an element which is a patient problem, with occurrences of individual problems reported by the patient. Each occurrence of a problem can be listed according to ICD-9 code and/or corresponding description. Each list is presented in its entirety. That is, the list is unfiltered and/or is not a subset. For example, the patient problem list is presented with all the reported problems to a healthcare practitioner. Problems are typically only removed from the list after careful review by a healthcare practitioner, which typically indicates that the listed problem is in error or that the patient is no longer experiencing the problem.

The lists are typically ordered in reverse chronological order. For example, as new problems are reported by a patient, the new problems are added to the top of the patient problem list, e.g. formed with a default chronological order. The lists can be lengthy and are important sources of information to healthcare practitioners, who are generally expected in a standard of patient care to be aware of any relevant occurrence on the list in delivering care. For example, a healthcare practitioner is reasonably expected to be aware of chronic conditions, which may be highly relevant and are naturally aged to the bottom of the list.

Occurrences on each list can have different relevance to different healthcare practitioners, who can be involved in different aspects of patient care. For example, "Falls frequently" is less relevant to a radiologist, than "Diabetes mellitus", while the reverse may hold for an orthopedist. Furthermore, the relevance of any one occurrence relative to another occurrence on the list is difficult to identify with precision by any one practitioner. That is, a healthcare practitioner may have difficulty in precisely reordering each occurrence in the list.

One approach to facilitate understanding in a viewing of relevant occurrences first can be to rank or re-order the list according to importance defined using an algorithm. However, such an approach does not consider individual or institutional preferences.

Another approach to facilitate understanding in a viewing of occurrences can be to use machine learning in how healthcare practitioners would reorder the list. However, such an approach is typically mutually exclusive of the approach of ranking by importance according to an algorithm, and healthcare practitioners are typically severely constrained with time to reorder each list. That is, there is limited time by healthcare practitioners to provide electronic feedback, which includes re-ordered lists of each individual patient. The reordering may involve decisions of determining precisely a relevance of each occurrence to others in the list, which diverts time and attention away from actual delivery of patient care. Moreover, the use of machine learning involves time for training the machine on appropriate learning, such as for example, a learning phase.

WO 2012/031052 A2 discloses a method of transacting medical information which includes receiving medical information from medical sources, identifying, mapping, and consolidating the received medical information by a back-end medical processor, providing access to specific relevant data, based on a user's security privileges, within the identified, mapped, and consolidated medical information, based on user-specific functions or roles by a front-end medical processor, and generating user-customized processed medical information to a plurality of users.

US 2015/0242571 Al discloses system and method for problem list categorization and management in an electronic health or medical record including matching each entry in the list with an interface terminology concept, grouping related concepts together into one or more categories, and grouping entries into one or more nested sets of problems.

US 2016/0147946 Al discloses systems, methods, and apparatus provided to facilitate analysis, presentation, and comparison of clinical information. An example system includes a processor configured to provide a patient library interface. The interface displays a plurality of events along a patient timeline and a list of items for comparison to a clinical scenario.

US 2008/0189645 Al discloses a sliding tab tabbed pane user interface component implementable in an electronic device. The user interface component provides a user interface of the electronic device with items belonging to a plurality of different categories, each category being associated with a different sliding tab. The sliding tabs are arranged such that at least a portion of the items of at least two sliding tabs is visible.

US 8,214,380 Bl discloses a system and method for managing search results available from a network resource using a data feed incorporating a processor programmed to retrieve user unsorted search results matching search terms provided by a user, transmit the search results along with a user selectable sorting identifier over the network for display on a graphical user interface and further programmed to sort each search result according to the user selected sorting identifier, update the sorting status of each search result, and transmit for display on the graphical user interface the search results in accordance with the sorting status and the user selected sorting identifier.

US 7,676,469 Bl discloses a system and method for updating a source copy of an ordered list (the source list) comprising a plurality of list items according to modifications made to the order of the list items in a local copy of the ordered list (the local list). Each list item includes an order value.

Oracle's Fusion Middleware User's Guide for Oracle WebCenter Spaces page 64-18 discloses rearranging list columns on all instances of a List.

### SUMMARY OF THE INVENTION

Aspects described herein address the above-referenced problems and others. The following describes contextual list viewing with sparse feedback.

The invention is defined by the independent claims. Dependent claims represent beneficial embodiments.

The following describes the invention according to one aspect of the invention. The following steps relate to a system, a method and non-transitory computer-readable storage medium claims. Exemplary, a system is described, comprising: a ranking engine comprising a first processor configured to receive a list for a patient which includes a plurality of occurrences, compute a relevance score for each occurrence in the list, wherein the computed relevance score is according to a relevance scheme that maps relevance scores from a lexicon controlling the list to each of the plurality of occurrences; and a user interface comprising a second processor. The user interface is configured to display the list on a display device of a local computing device ordered by a presented computed relevance score, wherein each displayed occurrence of the plurality of occurrences includes a feedback indicator; receive feedback comprising an input for one displayed occurrence of the plurality of occurrences according to the feedback indicator which indicates the one displayed occurrence is to be displayed higher or lower in the list than a current position, wherein the input is a binary indicator. The user interface is further configured to compute a feedback relevance score according to the binary indicator and a set of rules, wherein the feedback comprises the feedback relevance score and the system further comprises a feedback database comprising electronic storage and one or more processors configured to receive and store the feedback. The ranking engine is further configured to compute an adjusted relevance score according to the feedback for at least one occurrence of the plurality of occurrences, wherein the ranking engine computes the adjusted relevance score according to a function of all the feedback in the feedback database for the at least one occurrence of the plurality of occurrences in the list, the function including an average or mean of the feedback relevance scores and the function including only the last N feedback relevance scores; and the presented computed relevance score is the adjusted relevance score, where the feedback has been received, or the computed relevance score, where no or insufficient feedback has been received.

In a further aspect of the invention, a computer program product comprising instructions is described, wherein the instructions cause the computer to carry out the steps of the method of the present invention, when the program is executed by a computer.

These and other aspects of the invention will be apparent from and elucidated with reference to the embodiment(s) described hereinafter.

### BRIEF DESCRIPTION

The invention may take form in various components and arrangements of components, and in various steps and arrangements of steps.

### DETAILED DESCRIPTION OF EMBODIMENTS

An embodiment of a system 100 configured for contextual list viewing with sparse feedback (e.g., a contextual list viewing with sparse feedback system) is disclosed. A ranking engine 110 in response to a request 112 from a user receives or retrieves a list 114, such as a patient problem list, a list of patient medications, a surgical history list, a list of lab values, and the like. The request 112 includes identification of the list and the user requesting the list 114. The list 114 can be received from an EMR or other patient medical repository. The list 114, L, includes a plurality of occurrences, Lᵢ. In some optional embodiments, the occurrences in the list 114 include a time/date stamp, which indicates when each occurrence was entered into the list 114. In some optional embodiments, the occurrences in the list 114 include an identifier of the user that entered the occurrence.

For illustration purposes, the ranking engine 110 computes a relevance score for each occurrence, Lᵢ, according to a relevance scheme 116. A relevance scheme 116 includes a mapping between occurrences in the controlling lexicon and computed relevance scores. For example, ICD-9 codes of a patient problem list can be mapped to relevance scores represented as an interval [0,1], where higher values in the interval indicate greater relevance. The mappings of the relevance scheme 116 can be constructed from medical expert opinions, literature, data analysis, and the like. In some embodiments, the relevance scheme 116 is selected by a context manager 120 according to a user context 122.

The ranking engine 110 adjusts the computed relevance score according to feedback stored in a feedback database 124. The feedback can be modified or weighted by the context manager 120 according to the user context 122. In some embodiments, the user context 122 of the user requesting the list 114 is used to select and/or weight feedback. In some embodiments, the user context 122 of the user requesting the list 114 and the user context 122 of each user providing corresponding feedback is used to select and/or weight the feedback. In some embodiments, the user context 122 includes clinical context of a patient being care for at a time of the request or the feedback.

For illustration purposes, the following embodiment is provided. A user interface 130 presents a ranked list 132 with occurrences, Lᵢ, ordered by or ranked by a presented relevance score. The presented relevance score is the adjusted relevance score, where feedback 134 has been received, or the computed relevance score, where no or insufficient feedback 134 has been received. The presented ranked list 132 includes indicators 136 for which the feedback 134 can be indicated through an input, such as a single touch of a display 137. In some embodiments, the ranked list 132 is displayed again according to current feedback and prior feedback. That is, the ranking engine 110 receives the feedback 134 and re-computes the adjusted relevance score with the feedback 134 and that previously stored in the feedback database 124, and the user interfaces 130 presents the re-ranked list 132. In some embodiment, the re-ranking occurs with the next access of the list 114.

The context manager 120 can select a mapping for use by the ranking engine 110 from among a plurality of mappings of the relevance scheme 116 according to the user context 122. In some optional embodiments the relevance scheme can include mappings based on the identifiers of users entering occurrences and the user context 122.The user context 122 can include elements, such as healthcare specialty or service domain of the user, a clinical context, a healthcare role of the user, and/or combinations thereof. The healthcare specialty or service domain, such as Radiology, cardiology, ICU, oncology, etc., and healthcare role of the user, such as nurse, technician, resident, attending physician, etc., can be included in the request 112 or can be retrieved based on a user identifier from a user profile, security database, and the like. The clinical context, such as imaging study, bedside, discharge, etc., can be inferred by a location, type of examination scheduled, and the like. The clinical context can include an anatomical identification, such as identified from an imaging modality of a scheduled imaging study. The location of the user can be obtained from a user or local computing device 140, such as according to a global positioning system (GPS) component 148 or computer network location. The elements can be received from the user or local computing device 140 or other data stores and/or systems.

In some optional embodiments, each of the healthcare specialty or service domain of the user, the clinical context, or the healthcare role of the user can include a domain ontology 138, which can provide a hierarchical relationship between individual occurrences in the domain. For example, a hierarchical context of a physician (parent) healthcare role can include a resident physician (child), and an attending physician (child). A relevance scheme 116 exists for the physician (parent) and one for the attending physician (child) and not one for the resident (child). Thus, a hierarchical reasoning by the context manager 120 for a resident (child) may select a physician (parent) relevance scheme 116, while selecting the more specific relevance scheme 116 for an attending physician (child) over the physician (parent) for an attending physician. Another example includes radiology (parent) specialties, which can include subspecialties, such as by anatomy (children) and/or imaging modality (children): computed tomography (CT), magnetic resonance (MR), positron emission tomography (PET), single proton emission computed tomography (SPECT), ultrasound (US) and the like. In some instances, the clinical context according to the healthcare specialty or service domain of the user, the clinical context, or the healthcare role of the user with respect to the domain ontology 138 is such that healthcare practitioners more experienced and/or with better training for a given clinical and patient context receives more weight. For example, in caring for a critically ill patient in an ICU, feedback of an attending physician receives more weight than a critical care fellow. The critical care fellow receives more weight than a critical care intern. The critical care intern receives more weight than a medical student.

In addition, the elements can be inter-related, and a relevance scheme 116 determined from the combination based on a proximity of elements to an occurrence in the list 114. For example, a proximity can be derived between a clinical context of a brain MR exam and a healthcare role of a neurosurgeon with a selected relevance scheme 116.

The feedback database 124 electronically stores the feedback 134, which can be received from one or more local or user devices 140 for each list by the ranking engine 110. Examples of the feedback 134 as a structured stored feedback n-tuple can include (user identifier, occurrence identifier, presented relevance score, feedback relevance score, binary feedback, age of occurrence, patient clinical context), or (user identifier, occurrence identifier, presented relevance score, feedback relevance score, binary feedback). The user identifier can be related to the user role, user specialty, and/or combinations thereof, such as through a user profile. The occurrence identifier, Lᵢ, is an occurrence according to the lexicon, such as an ICD-9 code, a CPT code, and the like. The binary feedback is an indicator or value that indicates whether the feedback indicates that the occurrence identifier is to be listed higher or lower in relevance relative to a current position according to a presented relevance score for occurrences in the ranked list 132. The binary feedback can be indicated with binary values, such as 0 and 1, binary labels, such as "Up" and "Down", and the like. The age of occurrence, such as number of days, can be obtained from the date/time stamp according to the entry of the occurrence into the list.

In some optional embodiments the feedback database 124 can include a relationally structured format accessed by structured query language (SQL). In some embodiments, the feedback database 124 can include unstructured formats, such as storing contextual information of an imaging examination from a digital imaging and communication in medicine (DICOM) header of a study, which can include free text. In some embodiments, a combination of structured and unstructured database formats can be used.

The ranking engine 110 computes the adjusted relevance score according to a function of all the feedback in the feedback database 124 for the occurrence, Lᵢ in the list 114, the function including an average or mean of the feedback relevance scores Fᵢ. The function includes only the last N feedback relevance scores. For example, if feedback for a patient problem list for emphysema (ICD-9 code of 492) includes i=43 occurrences of feedback 134, then the 20 feedback relevance scores most recent in time can be used. In some embodiments, the feedback from the feedback database 124 includes a minimum number of occurrences of the feedback 134. That is, for Fᵢ, *i* > X, a predetermined threshold, for the ranking engine 110 to compute the adjusted relevancy score. In some embodiments, the function can include only the feedback relevance scores within a fixed time range or from a most recent entry to the list 114. For example, the feedback relevance scores within a most recent 90-daytime period can be used, or the feedback relevance scores received after a last update of the list 114. In some embodiments, the ranking engine 110 can use a decaying factor α to avoid abrupt changes in the feedback relevance scores between a presentation of the ranked list 132. For example, with feedback relevance scores temporally ordered by F₀, ..., F_{M}, the adjusted relevance score is the sum of Exp(α, i) × Fᵢ for 0 ≤ i ≤ M divided by the sum of Exp(α, i) for 0 ≤ i ≤ M.

In some otional embodiments, the ranking engine 110 uses hierarchical reasoning to select or supplement the feedback from the feedback database 124 that is used to compute the adjusted relevance score. The ranking engine 110 uses the list 114 that includes a domain ontology 138 with a supporting hierarchical structure, such as with ICD codes. For example, if an occurrence Lᵢ has not received feedback 134, then the feedback 134 for the parent of Lᵢ according to the domain ontology 138, can be used by the ranking engine 110 to adjust the relevancy score. The parent relationship can be used recursively using the domain hierarchy to obtain feedback from the occurrence to a root. In some embodiments, the ranking engine 110 can use feedback at different levels in the hierarchy until a sufficient number is received. The feedback according to occurrences at each level in the hierarchy can be weighted according to a distance from the occurrence, Lᵢ, to the level of the feedback. In some instances, the ranking engine 110 leverages sparse feedback across the domain ontology for a particular occurrence.

For illustration purposes, the context manager can weigh the feedback 134 in the feedback database 124 according to the user context 122. For a set of feedback relevance scores, F₀, ..., F_{M}, for an occurrence, Lᵢ, received from corresponding users, U₀, ..., U_{M}, weights, w₀, ..., w_{M}, can be computed according to a proximity of the user context 122 of each user providing feedback to user context 122 of the user requesting the list 114. Thus, the ranking engine 110 computes the adjusted relevance score using a set of weighted feedback relevance scores, F₀ x w₀, ..., F_{M} x w_{M}. For example, the weight for feedback from a neuroradiologist, wᵢ, may be greater for a neurosurgeon viewing a problem list, than the weight for feedback from an x-ray technician, wⱼ, where wᵢ>wⱼ. The weight can be represented as a continuous number of a distance between the user context 122 of the feedback 134 and the user context 122 of the user requesting the list 114. The weight can be computed as an inverse of the distance. For example, the closer the user context 122 of the feedback 134 and the user context of the requesting user, the lower the distance, the higher the weight, where the weight is expressed as (1/D). In some instances, this weighting can be used with hierarchical reasoning of the user context 122 according to one or more of domain ontologies 138.

In some optional embodiments the context manager 120 can filter the feedback 134 according a set of rules of varying contextual granularity of the user context 122. In some embodiments, the feedback 134 filtered from the feedback database 124 according to a list 114, such as a patient problem list, is for a plurality of patients. In some embodiments, the feedback 134 filtered from the feedback database 124 according to a list 114, is for a single patient.

For example, filtering can include a fine granularity of filtered feedback 134 from the feedback database 124 according to the user context 122 of the user requesting the list 114 that filters for (user and anatomy and imaging modality). The user is filtered for the specific user matching the user requesting the list 114 with the feedback 134 from the feedback database 124, such as by user identification, and an anatomy and an imaging modality according to the clinical context are also filtered according to the match. The feedback used by the ranking engine 110 to compute the adjusted relevance score would then be limited to a set of feedback specific to the user, anatomy and imaging modality. A more coarse filter filters according to (user and anatomy). A further more coarse filter filters according only to (anatomy). A ladder of decreasing contextual granularity from (user, anatomy, modality) to (user, anatomy) to (anatomy) is established. The context manager 120 can filter records from the feedback database 124 at varying granularities to obtain a sufficient number of feedback for the ranking engine 110 to compute the adjusted relevance score. That is, if filtering at one level of granularity does not produce a number of feedback records, F₁, ..., Fₙ, that exceeds a predetermined threshold, the filter can be reapplied with a next decreased level of granularity until sufficient feedback is obtained.

Filtering at different granularities can include the clinical context with data obtained from DICOM headers, such as anatomy, protocol, and/or modality. For example, the granularities can be extended to include (user, anatomy, protocol, modality), (user, anatomy, modality), (user, anatomy) and (anatomy). In some instances, the filtering at different levels of granularity can provide for pooling of feedback between users, and/or pooling of feedback from more generalized clinical contexts and the like.

The ranking engine 110, the context manager 120 and the user interface 130 are suitably embodied by one or more configured processors, such as one or more processors 142 of the user or local computing device 140 and one or more processors 150 of a computer server 152. The configured processor(s) 142, 150 execute at least one computer readable instruction stored in computer readable storage medium, such as the memory 144 of the user or local computing device 140 or server 152, which excludes transitory medium and includes physical memory and/or other non-transitory medium to perform the disclosed relevance score computing, ranking, contextual determination, hierarchical reasoning, feedback and display techniques. The configured processor may also execute one or more computer readable instructions carried by a carrier wave, a signal or other transitory medium. The user or local computing device 140 can comprise a workstation, laptop, tablet, smart phone, body worn computing device, combinations and the like. The server 152 can comprise one or more computer servers known in the art. The lines between components represented in the diagram represent communications paths, which can be wired or wireless.

The relevance scheme 116, feedback database 124 and the user context 122 are suitably embodied by computer storage media, such as local disk, cloud storage, remote storage, and the like, accessed by one or more configured computer processors. The user or local computing device 140 includes the display device 137, such as a computer display, projector, body worn display, and the like, and one or more input devices 146, such as a mouse, keyboard, microphone, touch or gesture interface, and the like. The local or user computing device 140 includes processors 142, such as a digital processor, a microprocessor, an electronic processor, an optical processor, a multi-processor, a distribution of processors including peer-to-peer or cooperatively operating processors, client-server arrangement of processors, and the like.

Referring to an embodiment, an example of a contextual list view of a patient problem list 200 with sparse feedback is disclosed. The patient problem list 200 is a presented list 114 that includes occurrences 210 of problems reported for a patient. For example, occurrences include "Falls frequently," "Aneurysm of anterior cerebral artery," and "Diabetes mellitus.".

Each occurrence 210 includes the indicators 136, which are illustrated as "[Down]," "[Up]. The indicators 136, such as two touch sensitive areas, two buttons, and the like, are used to indicate binary feedback in an input that the corresponding occurrence is to be re-ranked up, is more relevant, or is to be positioned higher in the list, or is to be re-ranked down, is less relevant, or is to be positioned lower in the presented list 132. The input can be a single input, such as a single touch, single gesture, single mouse click, and the like.

In some optional embodiments, the indicators 136 are sticky, so that if the user presses "Up" a second time, the indicator 136 is de-selected or non-indicated. In some embodiments, the indicators 136 interact like radio buttons such that if one is selected, the other is automatically de-selected. In some embodiments, once the indicator 136 is selected, the indicators 136 are removed for all occurrences until a next presentation of the ranked list 132. The view displaying the ranked list 132, such as the patient problem list 200, can include other visual aids, such as a scroll bar. That is, the view of the ranked list 132 can include a displayed portion of the ranked list 132, while the entire ranked list 132 remains accessible through the visual aid. The view displaying the ranked list 132 can include a position of an occurrence in the ranked list 132, such as numbered from 1 to N of a list of N occurrences, and the ranked list 132 is ranked according to the presented relevancy score.

In an example, rules that adjust relevance scores through feedback are disclosed. The example rules are formatted in a table according to the binary feedback or feedback indicators 136 and a position of the occurrence in the ranked list 132. The rules provide a mapping from a position of an occurrence in the ranked list to a feedback relevance score as a function of presented relevance scores of one or more occurrences.

In some optional embodiments, the example rules are generalized according to a top X occurrences of the presented relevance score and a bottom Y occurrences of the presented relevance score, where X and Y are integers. For example, X and Y can be 5. X and Y can be the same or different. In some embodiments X and Y can be based on the length of the list 114, that is, the number of occurrences, Z, in the list 114. For example, where Z < 10, the top Z/4 can be used rounded down to a nearest integer.

A first set of example rules address a feedback indicator 136 value of "Up" with the feedback for an occurrence in the top X presented occurrences. Two alternative rules are presented. A first rule includes the feedback relevance score computed as the sum of highest presented relevance score plus a constant. For example, with the top 5 presented relevance scores of (.90, .89, .89, .83, .72), and a constant of .02, the feedback relevance score is computed as .92. A second rule computes the feedback relevance score as a square root of the highest presented relevance score. Using the above set of top 5 scores, the feedback relevance score is computed as the SQRT (.90) =.95.

A second set of example rules address a feedback indicator 136 value of "Up" with the feedback for an occurrence not in the top X presented relevance score occurrences. The position of the occurrence with the binary feedback is lower in the list than the top X presented relevance score occurrences. The example rule of a computed feedback relevance score is an average of the presented relevance scores of the top X occurrences. Using the above example set of top 5 presented relevance score occurrences, the average of (.90, .89, .89, .83, .72) = .85.

A third set of example rules address a feedback indicator 136 value of "Down" with the feedback for an occurrence among in the top X presented relevance occurrences. An example rule computes the feedback relevance score as an average or a median of presented relevance scores for all presented relevance scores but the top X occurrences. That is, a set of presented relevance scores for computing the average includes those presented relevance scores for the ranked list 132 excluding the top X occurrences of the presented relevance scores. Another example alternative rule computes the feedback relevance score as an average or a median of presented relevance scores for the bottom Y presented relevance scores.

A fourth set of example rules address a feedback indicator 136 value of "Down" with the feedback for an occurrence not among in the top X occurrences. An example rule computes the feedback relevance score as a constant, such as zero.

An illustrative embodiment of a method of viewing a contextual list with sparse feedback is disclosed.

At 400, the list 114 is received. The list 114 can be received in response to the request 112 from the user or local computer device 140. The request can include the user context 122 or data, such as user identification, GPS location, and the like, which is used to identify the user context 122 according to a profile or other data stores.

At 410, a relevance score is computed for each occurrence of the list 114. The computed relevance score is computed according to a relevance scheme 116 that maps relevance scores for a lexicon controlling the list 114 to individual occurrences. The relevance scheme 116 can be selected according to the user context 122 by a context manager 120. The selection can include hierarchical reasoning and/or a proximity measurement between the user context 122 and the selected relevance scheme 116.

At 420, an adjusted relevance score is computed according to feedback stored in a feedback database 124 for the list 114 by the ranking engine 110. The feedback can be filtered, weighted, and/or supplemented by the context manager 120 according to the user context 122 of the user requesting the list 114 and the user context 122 of a user that provided the feedback 134 stored in the feedback database 124. The filtering, weighting, and/or supplementing can use hierarchical reasoning according to the domain ontology, which establishes relationships between occurrences of the elements of the user context 122. The filtering, weighting, and/or supplementing can use a distance measurement between elements of the user context 122 of the user requesting the list 114 and the user context 122 of a user that provided the feedback 134 stored in the feedback database 124.

At 430, the list 114 is presented as the ranked list 132, ordered by a presented relevance score. The presented relevance score for each occurrence is the adjusted relevance score if sufficient feedback is present in the feedback database 124. Otherwise, the presented relevance score is the computed relevance score. That is, if the feedback in the feedback database 124 is null or insufficient (does not exceed a predetermined threshold), the presented relevance score is the computed relevance score, computed at 410, otherwise is the adjusted relevance score, computed at 420. The presented ranked list 132 is displayed on the display device 137 of the user or local computing device 140 and includes feedback indicators 136 for each occurrence of the ranked list 132.

At 440, feedback 134 is received for one occurrence of the presented ranked list 132. The feedback 134 includes a binary indicator or value, which indicates that the corresponding occurrence is to be ranked higher or lower relative to the entire. A feedback relevance score is computed according to a set of rules, which is included in the feedback and stored in the feedback database 124.

The above may be implemented by way of computer readable instructions, encoded or embedded on computer readable storage medium, which, when executed by a computer processor(s), cause the processor(s) to carry out the described acts. Additionally or alternatively, at least one of the computer readable instructions is carried by a signal, carrier wave or other transitory medium.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims.

## Claims

1. A system, comprising:
a ranking engine comprising a first processor configured to receive a list for a patient which includes a plurality of occurrences, compute a relevance score for each occurrence in the list, wherein the computed relevance score is according to a relevance scheme that maps relevance scores from a lexicon controlling the list to each of the plurality of occurrences; and
a user interface comprising a second processor configured to:
display the list on a display device of a local computing device ordered by a presented computed relevance score, wherein each displayed occurrence of the plurality of occurrences includes a feedback indicator;
receive feedback comprising an input for one displayed occurrence of the plurality of occurrences according to the feedback indicator which indicates the one displayed occurrence is to be displayed higher or lower in the list than a current position, wherein the input is a binary indicator; and
wherein the user interface is further configured to compute a feedback relevance score according to the binary indicator and a set of rules, wherein the feedback comprises the feedback relevance score and
the system further comprises a feedback database comprising electronic storage and one or more processors configured to receive and store the feedback, and
wherein the ranking engine is further configured to compute an adjusted relevance score according to the feedback for at least one occurrence of the plurality of occurrences,
wherein the ranking engine computes the adjusted relevance score according to a function of all the feedback in the feedback database for the at least one occurrence of the plurality of occurrences in the list, the function including an average or mean of the feedback relevance scores and the function including only the last N feedback relevance scores; and
the presented computed relevance score is the adjusted relevance score, where the feedback has been received, or the computed relevance score, where no or insufficient feedback has been received.

2. The system according to claim 1, wherein the list is selected from a group comprising of a patient problem list, a patient medication list, a patient surgical history list, and a list of lab values for a patient.

3. The system according to any one of claims 1-2, further comprising:
a context manager comprising one or more processors configured to select the relevance scheme from a plurality of relevance schemes according to a user context of the local computing device displaying the list.

4. The system according to claim 3, wherein the context manager is further configured to select the feedback from the feedback database according to the user context of the local computing device displaying the list, wherein the feedback includes a user context of a user that provided the feedback.

5. The system according to either one of claims 3-4, wherein the context manager is further configured to weight the feedback from the feedback database according to the user context of the local computing device displaying the list.

6. The system according to any one of claims 3-5, wherein the user context comprises at least one selected from a group comprising of a healthcare practitioner role, a healthcare practitioner specialty, and a clinical context.

7. A method, comprising:
receiving a list for a patient which includes a plurality of occurrences;
computing a relevance score for each occurrence in the list, and wherein the computed relevance score is according to a relevance scheme that maps relevance scores from a lexicon controlling the list to each of the plurality of occurrences; and
displaying the list on a display device of a local computing device ordered by a presented computed relevance score, wherein each displayed occurrence of the plurality of occurrences includes a feedback indicator;
receiving feedback comprising an input for one displayed occurrence of the plurality of occurrences according to the feedback indicator which indicates the one displayed occurrence is to be displayed higher or lower in the list than a current position, wherein the input is a binary indicator;
computing a feedback relevance score according to the binary indicator and a set of rules, wherein the feedback comprises the feedback relevance score and storing the feedback in a feedback database;
computing an adjusted relevance score according to the feedback for at least one occurrence of the plurality of occurrences, wherein the adjusted relevance score is computed according to a function of all the feedback in the feedback database for the at least one occurrence of the plurality of occurrences in the list, the function including an average or mean of the feedback relevance scores and the function including only the last N feedback relevance scores; and wherein
the presented computed relevance score is the adjusted relevance score, where the feedback has been received, or the computed relevance score, where no or insufficient feedback has been received.

8. The method according to claim 7, wherein the list is selected from the group comprising of a patient problem list, a patient medication list, a patient surgical history list, and a list of lab values for a patient.

9. The method according to any one of claims 7-8, wherein computing the relevance score includes: selecting the relevance scheme from a plurality of relevance schemes according to a user context of the local computing device displaying the list.

10. The method according to any one of claims 7-9, wherein computing the adjusted relevance score includes: selecting the feedback from the feedback database according to the user context of the local computing device displaying the list, wherein the feedback includes a user context of a user that provided the feedback.

11. The method according to any one of claims 7-10, wherein computing the adjusted relevance score includes: weighting the feedback from the feedback database according to the user context of the local computing device displaying the list, wherein the feedback includes a user context of a user that provided the feedback.

12. A non-transitory computer-readable storage medium carrying instructions which controls one or more processors to:
receive a list for a patient which includes a plurality of occurrences;
compute a relevance score for each occurrence in the list, and wherein the computed relevance score is according to a relevance scheme that maps relevance scores from a lexicon controlling the list to each of the plurality of occurrences; and
display the list on a display device of a local computing device ordered by a presented computed relevance score, wherein each displayed occurrence of the plurality of occurrences includes a feedback indicator;
receive feedback comprising an input for one displayed occurrence of the plurality of occurrences according to the feedback indicator which indicates the one displayed occurrence is to be displayed higher or lower in the list than a current position, wherein the input is a binary indicator;
compute a feedback relevance score according to the binary indicator and a set of rules, wherein the feedback comprises the feedback relevance score, the feedback stored in a feedback database;
compute an adjusted relevance score according to the feedback for at least one occurrence of the plurality of occurrences, wherein the adjusted relevance score is computed according to a function of all the feedback in the feedback database for the at least one occurrence of the plurality of occurrences in the list, the function including an average or mean of the feedback relevance scores and the function including only the last N feedback relevance scores; and wherein the presented computed relevance score is the adjusted relevance score, where the feedback has been received, or the computed relevance score, where no or insufficient feedback has been received.

13. A computer program product comprising instructions which, when the program is executed by a computer, cause the computer to carry out the steps of the method of any one of claims 7-11.

## Patentansprüche

1. System, umfassend:
eine Rankingmaschine, die einen ersten Prozessor umfasst, der konfiguriert ist, um eine Liste für einen Patienten zu empfangen, die eine Vielzahl von Vorkommen beinhaltet, und eine Relevanzbewertung für jedes Vorkommen in der Liste zu berechnen, wobei sich die berechnete Relevanzbewertung nach einem Relevanzschema richtet, das Relevanzbewertungen aus einem Lexikon, das die Liste steuert, jedem der Vielzahl von Vorkommen zuordnet; und
eine Benutzerschnittstelle, die einen zweiten Prozessor umfasst, der konfiguriert ist, um:
die Liste auf einer Anzeigevorrichtung einer lokalen Rechenvorrichtung, geordnet nach einer dargestellten berechneten Relevanzbewertung anzuzeigen, wobei jedes angezeigte Vorkommen der Vielzahl von Vorkommen einen Rückmeldungsindikator beinhaltet;
eine Rückmeldung zu empfangen, die eine Eingabe für ein angezeigtes Vorkommen der Vielzahl von Vorkommen gemäß dem Rückmeldungsindikator umfasst, der angibt, dass die ein angezeigtes Vorkommen weiter oben oder weiter unten in der Liste als eine aktuelle Position anzuzeigen ist, wobei die Eingabe ein binärer Indikator ist; und
wobei die Benutzerschnittstelle weiter konfiguriert ist, um eine Rückmeldungsrelevanzbewertung gemäß dem binären Indikator und einem Satz von Regeln zu berechnen, wobei die Rückmeldung die Rückmeldungsrelevanzbewertung umfasst und
das System weiter eine Rückmeldungsdatenbank umfasst, die einen elektronischen Speicher und einen oder mehrere Prozessoren umfasst, die konfiguriert sind, um die Rückmeldung zu empfangen und zu speichern, und wobei die Rankingmaschine weiter konfiguriert ist, um eine angepasste Relevanzbewertung gemäß der Rückmeldung für mindestens ein Vorkommen der Vielzahl von Vorkommen zu berechnen,
wobei die Rankingmaschine die angepasste Relevanzbewertung gemäß einer Funktion von jeder Rückmeldung in der Rückmeldungsdatenbank für das mindestens eine Vorkommen der Vielzahl von Vorkommen in der Liste berechnet, wobei die Funktion einen Durchschnitt oder Mittelwert der Rückmeldungsrelevanzbewertungen beinhaltet und die Funktion nur die letzten N Rückmeldungsrelevanzbewertungen beinhaltet; und
die präsentierte berechnete Relevanzbewertung die angepasste Relevanzbewertung, wenn die Rückmeldung empfangen worden ist, oder die berechnete Relevanzbewertung ist, wenn keine oder eine unzureichende Rückmeldung empfangen worden ist.

2. System nach Anspruch 1, wobei die Liste aus einer Gruppe ausgewählt ist, die eine Liste mit einem Patientenproblem, eine Liste mit einer Patientenmedikation, eine Liste mit der chirurgischen Vorgeschichte eines Patienten und eine Liste mit Laborwerten für einen Patienten umfasst.

3. System nach einem der Ansprüche 1-2, weiter umfassend:
einen Kontextmanager, der einen oder mehrere Prozessoren umfasst, die konfiguriert sind, um das Relevanzschema aus einer Vielzahl von Relevanzschemata gemäß einem Benutzerkontext der lokalen Rechenvorrichtung, die die Liste anzeigt, auszuwählen.

4. System nach Anspruch 3, wobei der Kontextmanager weiter konfiguriert ist, um die Rückmeldung aus der Rückmeldungsdatenbank gemäß dem Benutzerkontext der lokalen Rechenvorrichtung, die die Liste anzeigt, auszuwählen, wobei die Rückmeldung einen Benutzerkontext eines Benutzers beinhaltet, der die Rückmeldung bereitgestellt hat.

5. System nach einem der Ansprüche 3-4, wobei der Kontextmanager weiter konfiguriert ist, um die Rückmeldung aus der Rückmeldungsdatenbank gemäß dem Benutzerkontext der lokalen Rechenvorrichtung, die die Liste anzeigt, zu gewichten.

6. System nach einem der Ansprüche 3-5, wobei der Benutzerkontext mindestens eines umfasst, ausgewählt aus einer Gruppe, die eine Rolle des medizinischen Fachpersonals, ein Spezialgebiet des medizinischen Fachpersonals und einen klinischen Kontext umfasst.

7. Verfahren, umfassend:
Empfangen einer Liste für einen Patienten, die eine Vielzahl von Vorkommen beinhaltet;
Berechnen einer Relevanzbewertung für jedes Vorkommen in der Liste, und wobei sich die berechnete Relevanzbewertung nach einem Relevanzschema richtet, das Relevanzbewertungen aus einem Lexikon, das die Liste steuert, jedem der Vielzahl von Vorkommen zuordnet; und
Anzeigen der Liste auf einer Anzeigevorrichtung einer lokalen Rechenvorrichtung, geordnet nach einer dargestellten berechneten Relevanzbewertung, wobei jedes angezeigte Vorkommen der Vielzahl von Vorkommen einen Rückmeldungsindikator beinhaltet;
Empfangen einer Rückmeldung, die eine Eingabe für ein angezeigtes Vorkommen der Vielzahl von Vorkommen gemäß dem Rückmeldungsindikator umfasst, der angibt, dass das eine angezeigte Vorkommen weiter oben oder weiter unten in der Liste als eine aktuelle Position anzuzeigen ist, wobei die Eingabe ein binärer Indikator ist;
Berechnen einer Rückmeldungsrelevanzbewertung gemäß dem binären Indikator und einem Satz von Regeln, wobei die Rückmeldung die Rückmeldungsrelevanzbewertung und Speichern der Rückmeldung in einer Rückmeldungsdatenbank umfasst;
Berechnen einer angepassten Relevanzbewertung gemäß der Rückmeldung für mindestens ein Vorkommen der Vielzahl von Vorkommen, wobei die angepasste Relevanzbewertung gemäß einer Funktion von jeder Rückmeldung in der Rückmeldungsdatenbank für das mindestens eine Vorkommen der Vielzahl von Vorkommen in der Liste berechnet wird,
wobei die Funktion einen Durchschnitt oder Mittelwert der Rückmeldungsrelevanzbewertungen beinhaltet und die Funktion nur die letzten N Rückmeldungsrelevanzbewertungen beinhaltet; und wobei
die präsentierte berechnete Relevanzbewertung die angepasste Relevanzbewertung, wenn die Rückmeldung empfangen worden ist, oder die berechnete Relevanzbewertung ist, wenn keine oder eine unzureichende Rückmeldung empfangen worden ist.

8. Verfahren nach Anspruch 7, wobei die Liste aus der Gruppe ausgewählt ist, die eine Liste mit einem Patientenproblem, eine Liste mit einer Patientenmedikation, eine Liste mit der chirurgischen Vorgeschichte eines Patienten und eine Liste mit Laborwerten für einen Patienten umfasst.

9. Verfahren nach einem der Ansprüche 7-8, wobei die Berechnung der Relevanzbewertung beinhaltet: Auswählen des Relevanzschemas aus einer Vielzahl von Relevanzschemata gemäß einem Benutzerkontext der lokalen Rechenvorrichtung, die die Liste anzeigt.

10. Verfahren nach einem der Ansprüche 7-9, wobei das Berechnen der angepassten Relevanzbewertung das Auswählen der Rückmeldung aus der Rückmeldungsdatenbank gemäß dem Benutzerkontext der lokalen Rechenvorrichtung, die die Liste anzeigt, beinhaltet, wobei die Rückmeldung einen Benutzerkontext eines Benutzers beinhaltet, der die Rückmeldung bereitgestellt hat.

11. Verfahren nach einem der Ansprüche 7-10, wobei das Berechnen der angepassten Relevanzbewertung beinhaltet: Gewichten der Rückmeldung aus der Rückmeldungsdatenbank gemäß dem Benutzerkontext der lokalen Rechenvorrichtung, die die Liste anzeigt, wobei die Rückmeldung einen Benutzerkontext eines Benutzers beinhaltet, der die Rückmeldung bereitgestellt hat.

12. Nichtflüchtiges, computerlesbares Speichermedium, das Anweisungen trägt, die einen oder mehrere Prozessoren steuern, um:
eine Liste für einen Patienten, die eine Vielzahl von Vorkommen beinhaltet, zu empfangen;
eine Relevanzbewertung für jedes Vorkommen in der Liste zu berechnen, und wobei sich die berechnete Relevanzbewertung nach einem Relevanzschema richtet, das Relevanzbewertungen aus einem Lexikon, das die Liste steuert, jedem der Vielzahl von Vorkommen zuordnet; und
die Liste auf einer Anzeigevorrichtung einer lokalen Rechenvorrichtung, geordnet nach einer dargestellten berechneten Relevanzbewertung anzuzeigen, wobei jedes angezeigte Vorkommen der Vielzahl von Vorkommen einen Rückmeldungsindikator beinhaltet;
eine Rückmeldung zu empfangen, die eine Eingabe für ein angezeigtes Vorkommen der Vielzahl von Vorkommen gemäß dem Rückmeldungsindikator umfasst, der angibt, dass das eine angezeigte Vorkommen weiter oben oder weiter unten in der Liste als eine aktuelle Position anzuzeigen ist, wobei die Eingabe ein binärer Indikator ist;
eine Rückmeldungsrelevanzbewertung gemäß dem binären Indikator und einem Satz von Regeln zu berechnen, wobei die Rückmeldung die Rückmeldungsrelevanzbewertung, wobei die Rückmeldung in einer Rückmeldungsdatenbank gespeichert wird, umfasst;
eine angepasste Relevanzbewertung gemäß der Rückmeldung für mindestens ein Vorkommen der Vielzahl von Vorkommen zu berechnen, wobei die angepasste Relevanzbewertung gemäß einer Funktion von jeder Rückmeldung in der Rückmeldungsdatenbank für das mindestens eine Vorkommen der Vielzahl von Vorkommen in der Liste berechnet wird,
wobei die Funktion einen Durchschnitt oder Mittelwert der Rückmeldungsrelevanzbewertungen beinhaltet und die Funktion nur die letzten N Rückmeldungsrelevanzbewertungen beinhaltet; und wobei
die präsentierte berechnete Relevanzbewertung die angepasste Relevanzbewertung, wenn die Rückmeldung empfangen worden ist, oder die berechnete Relevanzbewertung ist, wenn keine oder eine unzureichende Rückmeldung empfangen worden ist.

13. Computerprogrammprodukt, das Anweisungen umfasst, die, wenn das Programm von einem Computer ausgeführt wird, den Computer veranlassen, die Schritte des Verfahrens nach einem der Ansprüche 7-11 auszuführen.

## Revendications

1. Système, comprenant :
un moteur de classement comprenant un premier processeur configuré pour recevoir une liste pour un patient qui inclut une pluralité d'occurrences, calculer un score de pertinence pour chaque occurrence dans la liste, dans lequel le score de pertinence calculé est conforme à un schéma de pertinence qui mappe les scores de pertinence d'un lexique commandant la liste sur chacune de la pluralité d'occurrences ; et
une interface utilisateur comprenant un second processeur configuré pour :
afficher la liste sur un dispositif d'affichage d'un dispositif informatique local classée selon un score de pertinence calculé présenté, dans lequel chaque occurrence affichée de la pluralité d'occurrences inclut un indicateur de retour ;
recevoir un retour comprenant une entrée pour une occurrence affichée de la pluralité d'occurrences selon l'indicateur de retour qui indique que l'occurrence affichée est à afficher plus haut ou plus bas dans la liste qu'une position actuelle, dans lequel l'entrée est un indicateur binaire ; et
dans lequel l'interface utilisateur est en outre configurée pour calculer un score de pertinence de retour en fonction de l'indicateur binaire et d'un ensemble de règles, dans lequel le retour comprend le score de pertinence de retour et
le système comprend en outre une base de données de retours comprenant un stockage électronique et un ou plusieurs processeurs configurés pour recevoir et stocker les retours, et dans lequel le moteur de classement est en outre configuré pour calculer un score de pertinence ajusté en fonction des retours pour au moins une occurrence de la pluralité d'occurrences,
dans lequel le moteur de classement calcule le score de pertinence ajusté selon une fonction de tous les retours dans la base de données de retours pour la au moins une occurrence de la pluralité d'occurrences dans la liste, la fonction incluant une moyenne des scores de pertinence du retour et la fonction incluant uniquement les N derniers scores de pertinence de retours ; et
le score de pertinence calculé présenté est le score de pertinence ajusté, lorsque le retour a été reçu, ou le score de pertinence calculé, lorsque le retour n'a pas été reçu ou qu'il est insuffisant.

2. Système selon la revendication 1, dans lequel la liste est sélectionnée dans un groupe comprenant une liste de problèmes de patient, une liste de médicaments de patient, une liste d'antécédents chirurgicaux de patient et une liste de valeurs de laboratoire pour un patient.

3. Système selon l'une quelconque des revendications 1-2, comprenant en outre :
un gestionnaire de contexte comprenant un ou plusieurs processeurs configurés pour sélectionner le schéma de pertinence parmi une pluralité de schémas de pertinence en fonction d'un contexte utilisateur du dispositif informatique local affichant la liste.

4. Système selon la revendication 3, dans lequel le gestionnaire de contexte est en outre configuré pour sélectionner le retour à partir de la base de données de retours en fonction du contexte utilisateur du dispositif informatique local affichant la liste, dans lequel le retour inclut un contexte utilisateur d'un utilisateur ayant fourni le retour.

5. Système selon l'une ou l'autre des revendications 3-4, dans lequel le gestionnaire de contexte est en outre configuré pour pondérer le retour provenant de la base de données de retours en fonction du contexte utilisateur du dispositif informatique local affichant la liste.

6. Système selon l'une quelconque des revendications 3-5, dans lequel le contexte utilisateur comprend au moins un élément sélectionné dans un groupe comprenant un rôle de praticien de santé, une spécialité de praticien de santé et un contexte clinique.

7. Procédé, comprenant :
la réception d'une liste pour un patient qui inclut une pluralité d'occurrences ;
le calcul d'un score de pertinence pour chaque occurrence dans la liste, et dans lequel le score de pertinence calculé est conforme à un schéma de pertinence qui mappe les scores de pertinence d'un lexique commandant la liste sur chacune de la pluralité d'occurrences ; et
l'affichage de la liste sur un dispositif d'affichage d'un dispositif informatique local classée selon un score de pertinence calculé présenté, dans lequel chaque occurrence affichée de la pluralité d'occurrences inclut un indicateur de retour ;
la réception d'un retour comprenant une entrée pour une occurrence affichée de la pluralité d'occurrences selon l'indicateur de retour qui indique que l'occurrence affichée est à afficher plus haut ou plus bas dans la liste qu'une position actuelle, dans lequel l'entrée est un indicateur binaire ;
le calcul d'un score de pertinence de retour en fonction de l'indicateur binaire et d'un ensemble de règles, dans lequel le retour comprend le score de pertinence de retour et le stockage du retour dans une base de données de retours ;
le calcul d'un score de pertinence ajusté en fonction du retour pour au moins une occurrence de la pluralité d'occurrences, dans lequel le score de pertinence ajusté est calculé en fonction d'une fonction de tous les retours dans la base de données de retours pour la au moins une occurrence de la pluralité d'occurrences dans la liste,
la fonction incluant une moyenne des scores de pertinence de retour et la fonction incluant uniquement les N derniers scores de pertinence de retour ; et dans lequel
le score de pertinence calculé présenté est le score de pertinence ajusté, lorsque le retour a été reçu, ou le score de pertinence calculé, lorsque le retour n'a pas été reçu ou qu'il est insuffisant.

8. Procédé selon la revendication 7, dans lequel la liste est sélectionnée dans le groupe comprenant une liste de problèmes de patient, une liste de médicaments de patient, une liste d'antécédents chirurgicaux de patient et une liste de valeurs de laboratoire pour un patient.

9. Procédé selon l'une quelconque des revendications 7-8, dans lequel le calcul du score de pertinence inclut : la sélection du schéma de pertinence parmi une pluralité de schémas de pertinence en fonction d'un contexte utilisateur du dispositif informatique local affichant la liste.

10. Procédé selon l'une quelconque des revendications 7-9, dans lequel le calcul du score de pertinence ajusté inclut : la sélection du retour à partir de la base de données de retours en fonction du contexte utilisateur du dispositif informatique local affichant la liste, dans lequel le retour inclut un contexte utilisateur d'un utilisateur ayant fourni le retour.

11. Procédé selon l'une quelconque des revendications 7-10, dans lequel le calcul du score de pertinence ajusté inclut : la pondération du retour provenant de la base de données de retours en fonction du contexte utilisateur du dispositif informatique local affichant la liste, dans lequel le retour inclut un contexte utilisateur d'un utilisateur ayant fourni le retour.

12. Support de stockage non transitoire lisible par ordinateur contenant des instructions qui commandent un ou plusieurs processeurs pour :
recevoir une liste pour un patient qui inclut une pluralité d'occurrences ;
calculer un score de pertinence pour chaque occurrence dans la liste, et dans lequel le score de pertinence calculé est conforme à un schéma de pertinence qui mappe les scores de pertinence d'un lexique commandant la liste sur chacune de la pluralité d'occurrences ; et
afficher la liste sur un dispositif d'affichage d'un dispositif informatique local classée selon un score de pertinence calculé présenté, dans lequel chaque occurrence affichée de la pluralité d'occurrences inclut un indicateur de retour ;
recevoir un retour comprenant une entrée pour une occurrence affichée de la pluralité d'occurrences selon l'indicateur de retour qui indique que l'occurrence affichée est à afficher plus haut ou plus bas dans la liste qu'une position actuelle, dans lequel l'entrée est un indicateur binaire ;
calculer un score de pertinence de retour en fonction de l'indicateur binaire et d'un ensemble de règles, dans lequel le retour comprend le score de pertinence de retour, le retour stocké dans une base de données de retours ;
calculer un score de pertinence ajusté en fonction du retour pour au moins une occurrence de la pluralité d'occurrences, dans lequel le score de pertinence ajusté est calculé en fonction d'une fonction de tous les retours dans la base de données de retours pour la au moins une occurrence de la pluralité d'occurrences dans la liste,
la fonction incluant une moyenne des scores de pertinence de retour et la fonction incluant uniquement les N derniers scores de pertinence de retour ; et dans lequel
le score de pertinence calculé présenté est le score de pertinence ajusté, lorsque le retour a été reçu, ou le score de pertinence calculé, lorsque le retour n'a pas été reçu ou qu'il est insuffisant.

13. Produit de programme informatique comprenant des instructions qui, lorsque le programme est exécuté par un ordinateur, amènent l'ordinateur à réaliser les étapes du procédé selon l'une quelconque des revendications 7-11.
